# EUROPEAN PATENT SPECIFICATION

(11) **EP 0 449 295 B1**
(45) Date of publication and mention of the grant of the patent: **26.06.1996**
(21) Application number: 91105016.9
(22) Date of filing: 28.03.1991
(51) Int. Cl.: C07D 487/04, G01N 21/76

(54) **Novel quinoxalinone derivative**
Neues Chinoxalinonderivat
Nouveau dérivé de la quinoxalinone

(30) Priority: 30.03.1990 JP 83318/90
(43) Date of publication of application: 02.10.1991
(73) Proprietor: BIOSENSOR LABORATORIES CO., LTD., Toshima-ku, Tokyo (JP)
(72) Inventor: Yamaguchi, Masatoshi, Fukuoka-shi, Fukuoka-ken (JP)
(74) Representative: VOSSIUS & PARTNER

(56) References cited:
- CHEMICAL ABSTRACTS, vol. 67, no. 7, July31, 1967, Columbus, Ohio, USA; WHITE E.H. et al.: "The synthesis and chemilumin escence of an amino dewrivative and sulfur analog of luminol", page 2091, column 2, abstract-no. 21 890p.

## Description

This invention relates to a novel compound represented by the following general formula (I): 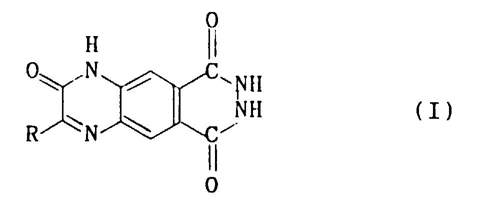 wherein R represents an arbitrary group.

More particularly, it relates to a quinoxalinone derivative represented by the general formula (I) which is useful as, for example, a luminescent labeling reagent or a reagent for determining hydrogen peroxide.

There have been reported several derivatization reagents to be used in the determination of α-keto acids by a spectroscopic or fluorescent method with the use of liquid chromatography.

C. Hemming and C. J. Gubler proposed to determine an α-keto acid by a spectroscopic method with the use of 2,4-dinitro-phenylhydrazine [refer to Anal. Biochem. 92 (1979) 31]. However this method gives only a poor detection sensitivity and no selectivity for α-keto acids. Regarding the determination of α-keto acids by a fluorescent method, on the other hand, there have been reported to use 4'-hydrazino-2-stilbazole [refer to T. Hirata, M. Kai, K. Kohashi and Y. Ohkura, J. Chromatogr., 226 (1981) 25] and o-phenylenediamine [refer to J.C. Liao, N.E. Hoffman, J.J. Barboriak and D.A. Roth, Clin. Chem., 23 (1977) 802; and T. Hayashi, H. Tsuchiya and H. Naruse, J. Chromatogr., 273 (1983) 245], each as a reagent. Although each of these methods gives a substantially high sensitivity, it is impossible thereby to determine an α-keto acid at a femtomol (fmol: 10⁻¹⁵ mol) level.

Formerly 1,2-diamino-4-5-dimethoxybenzene and 1,2-diamino-4,5-methylenedioxybenzene have been synthesized each as a fluorescent analysis reagent for α-keto acids having a high sensitivity and a high selectivity [the former reagent: refer to S. Hara, Y. Takemori, T. Iwata, M. Yamaguchi, M. Nakamura and Y. Ohkura, Anal. Chem. Acta, 172 (1985) 167; and S. Hara, M. Yamaguchi, M. Nakamura and Y. Ohkura, Chem. Pharm. Bull., 33 (1985) 3493; the latter one: refer to M. Nakamura, S. Hara, M. Yamaguchi, Y. Takemori and Y. Ohkura, Chem. Pharm. Bull., 35 (1987) 687]. It has been attempted to apply these reagents to liquid chromatography and thus succeeded in determining α-keto acids at a femtomol level. These reagents have been used for determining α-keto acids contained in human urine and serum [refer to S. Hara, Y. Takemori, M. Yamaguchi, N. Nakamura and Y. Ohkura, J. Chromatogr., 344 (1985) 33].

Recently, techniques for chemiluminescent detection have been introduced into liquid chromatographic analyses. As a result, there have been reported several reagents which react with amino acids, amine or carboxylic acids and thus covert these compounds into chemiluminescent derivatives [reagents for amino acids: refer to S.R. Spurlin and M.M. Cooper, Anal. Lett., 19 (1986) 2277; reagents for amines: refer to T. Kawasaki. M. Maeda and A. Tsuji, J. Chromatogr., 328 (1985) 121; reagents for carboxylic acids: refer to T. Kawasaki, M. Maeda and A. Tsuji, J. Chromatogr., 328 (1985) 121; H. Yuki, H. Azuma, M. Maeda and H. Kawasaki, Chem. Pham. Bull., 36 (1988) 1905; and H. Karatani, J. Takano, S. Morishita, M. Yoshida and M. Sato, Bunseki Kagaku, 38 (1989) 59].

As described above, attempts have been frequently made to determine α-keto acids by spectroscopic or fluorescent methods. However there has been reported hitherto no reagent required for sensitively and selectively determining a trace amount of an α-keto acid by utilizing chemiluminescence.

It is the object of the present invention to provide a compound which is useful as a luminescent labeling reagent or a reagent for determining hydrogen peroxide. This object could be achieved on the basis of the finding that intense chemiluminescence is generated by reacting a quinoxalinone derivative, which is obtained by the reaction of an α-keto acid with 4,5-diaminophthalhydrazide dihydrochloride (hereinafter referred to simply as 4,5-DPH·2HCl), with hydrogen peroxide in an alkaline polar medium in the presence of potassium hexacyanoferrate (III).

The above chemical reaction may be represented by the following formula:

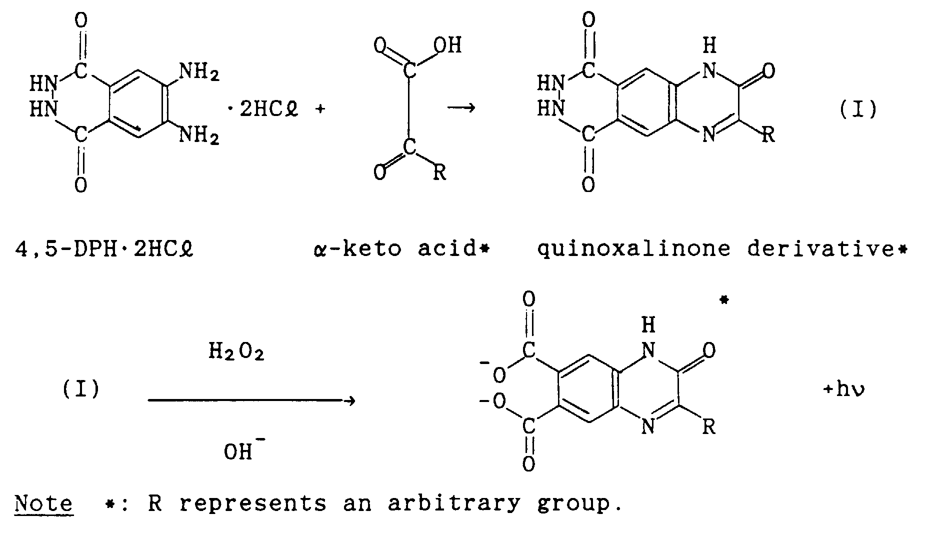

The quinoxalinone derivative of the general formula (I) obtained by the aforesaid chemical reaction is a novel compound which has never been described in any literature.

Hydrogen peroxide can be determined by using said compound per se as a reaction reagent and various substances can be sensitively detected by labeling said substance with the eforesaid compound as a luminescent labeling reagent.

The present invention relates to a quinoxalinone derivative represented by the following general formula (I): 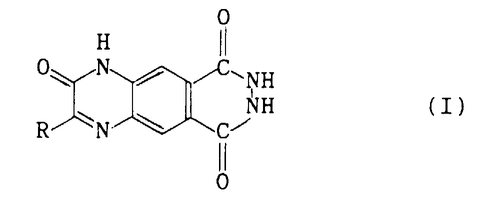 wherein R represents an arbitrary group.

As described above, R may be any group. Examples thereof include a hydrogen atom, an optionally substituted hydrocarbon group, an optionally substituted heterocyclic group, an amino group, a carboxyl group and a thiol group. In particular, those having functional groups capable of reacting with alkyl groups, phenyl-substituted alkyl groups, amino groups, carboxyl groups, thiol groups or alcoholic hydroxyl groups (for example, carboxyl groups, carbonyl halides, acid azides, succinimide esters, isothiocyanates, amino groups, maleimide groups and acid hydrazides) are preferable. Examples of functional groups capable of reacting with alcoholic hydroxyl groups are as follows:
-(CH₂)ₙ-X wherein n is 0 or an integer; and
X represents a group selected from among COOH, COCl, CON₂,

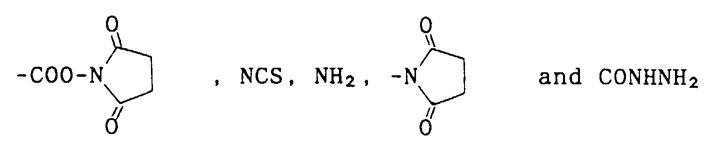

The quinoxalinone derivative of the present invention may be obtained by reacting an α-keto acid represented by the following general formula (II), wherein R represents an arbitrary group, with 4,5-diaminophthalhydrazide of the following formula (III):

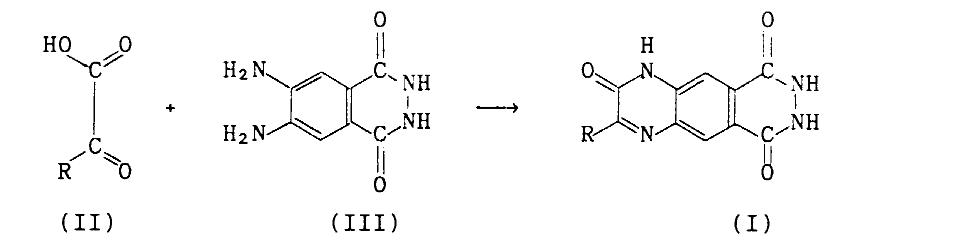

To further illustrate the present invention, and not by way of limitation, the following Examples will be given.

### [Examples 1 to 8]

4,5-diaminophthalhydrazide (0.3 mmol) and each α-keto acid (0.6 mmol) as specified in Table 1 were dissolved in a mixture of hydrochloric acid (2 mol) containing 1.2 mol of β-mercapto-ethanol (1.2 mol) and ethanol (1 : 1, v/v). The obtained mixture was heated in a water bath for 2 hours. After the completion of the heating, the mixture was cooled in ice/water. The precipitate thus formed was collected by filtering and washed with ethanol. Thus the corresponding quinoxalinone derivative was obtained. The yield ranged from 40 to 50% in the case of each α-keto acid.

Tables 1 and 2 show the physicochemical data of the quinoxalinone derivatives thus obtained.

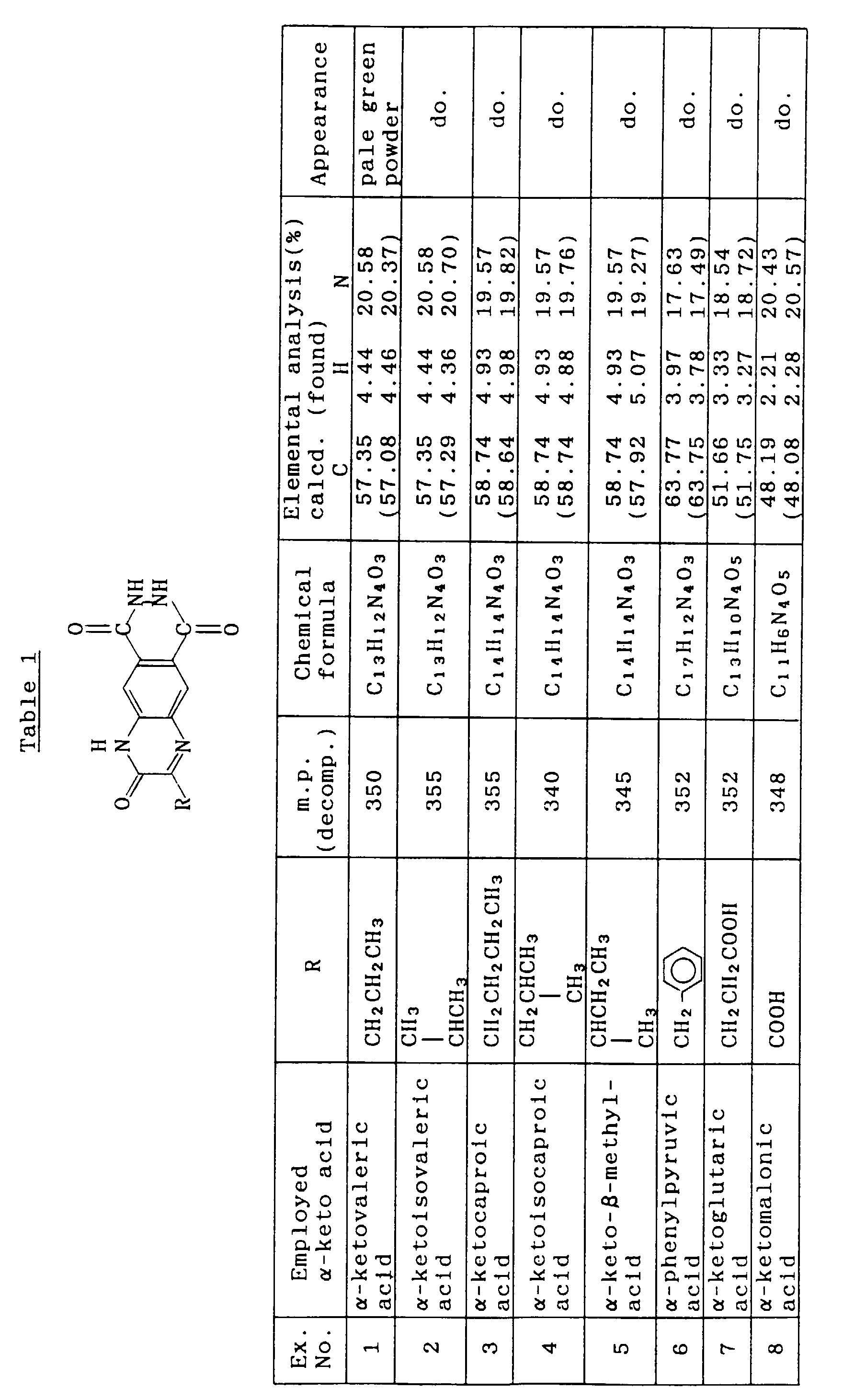

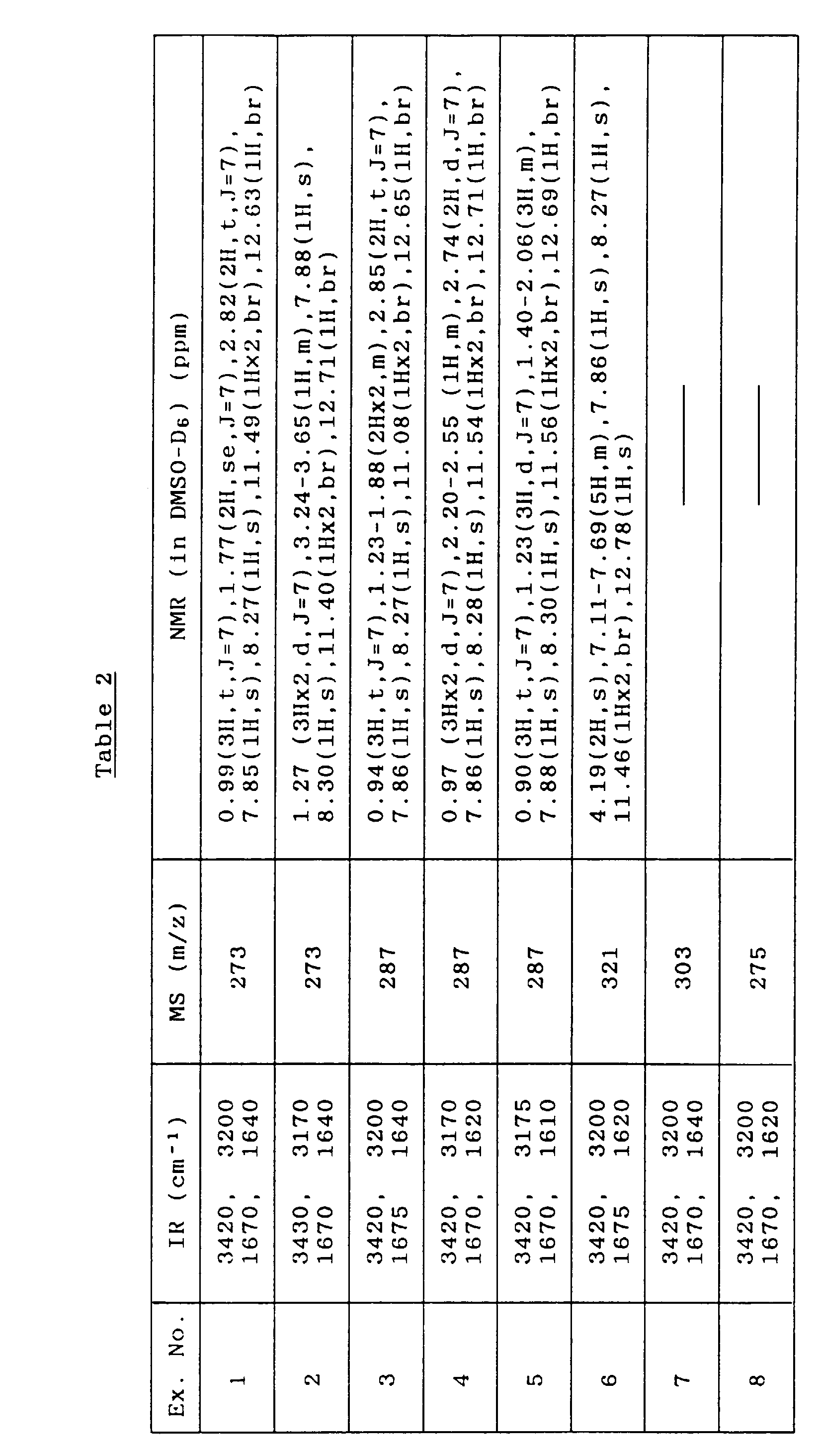

The quinoxalinone derivative of the present invention reacts with hydrogen peroxide in an alkaline solvent in the presence of potassium hexacyanoferrate (III), thus showing chemiluminescence. By utilizing this characteristic, the compound of the present invention can be applied to the following purposes.

### 1. Reagent for determining hydrogen peroxide

1) The compound of the present invention is available as a reagent for determining hydrogen peroxide formed by an enzymatic reaction (applicable to the measurement of the activity of an enzyme and to the assay of a substrate of an enzyme).
2) It is available as a reagent for determining hydrogen peroxide formed by a chemical reaction.

### 2. Luminescent labeling reagent

1) The compound of the present invention is available as a reagent for labeling, for example, proteins, amino acids, saccharides, lipids, vitamins, hormones and drugs. These substances to be determined, which have been labeled with the compound of the present invention, may be detected and determined by separating by high-performance liquid chromatography (HPLC) and then reacting with hydrogen peroxide.
2) It may be used for labeling, for example, antigens, antibodies, ligands, receptors, DNAs and RNAs. By using these substances labeled with the compound of the present invention, materials capable of undergoing a biochemical reaction with these substances can be assayed.

## Claims

1. A novel quinoxalinone derivative represented by the following general formula: 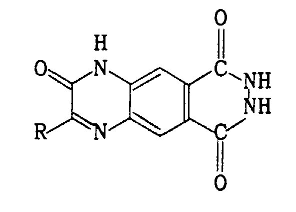 werein R represents an arbitrary group.

## Patentansprüche

1. Neues Chinoxalinonderivat der folgenden allgemeinen Formel: 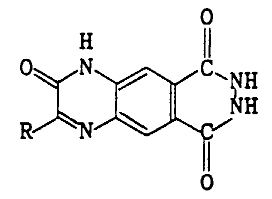 in der R einen beliebigen Rest darstellt.

## Revendications

1. Nouveau dérivé de la quinoxalinone, représenté par la formule générale suivante : 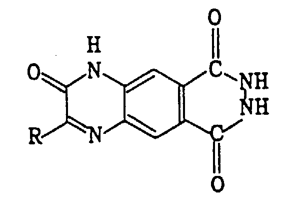 où R représente un radical arbitraire.
